# EUROPEAN PATENT APPLICATION

(11) **EP 4 094 768 A1**
(43) Date of publication of application: **30.11.2022**
(21) Application number: 21754172.1
(22) Date of filing: 29.01.2021
(51) Int. Cl.: A61K 33/00, A61K 45/00, A61P 9/08

(54) **VASODILATOR COMPOSITION, VASODILATOR COMPOSITION KIT, PHARMACEUTICAL COMPOSITION FOR DISEASES CAUSED BY BLOOD VESSEL CONSTRICTION OR OBSTRUCTIVE IMPAIRMENT, AND PHARMACEUTICAL COMPOSITION KIT FOR DISEASES CAUSED BY BLOOD VESSEL CONSTRICTION OR OBSTRUCTIVE IMPAIRMENT**

(30) Priority: 12.02.2020 JP 2020021195
(71) Applicant: Aichi Medical University, Aichi 480-1195 (JP)
(72) Inventor: HATAYAMA Naoyuki, Nagakute-shi, Aichi 480-1195 (JP); NAITO Munekazu, Nagakute-shi, Aichi 480-1195 (JP); HIRAI Shuichi, Nagakute-shi, Aichi 480-1195 (JP); FUKUSHIGE Kaori, Nagakute-shi, Aichi 480-1195 (JP)
(74) Representative: Hoffmann Eitle
(86) International application number: PCT/JP2021/003258
(87) International publication number: WO 2021/161811

(57) **Abstract**

The present invention provides a vasodilator composition capable of adjusting a dosage of nitric oxide (NO). A vasodilator composition of the present invention includes microbubbles, wherein the microbubbles contain NO as a gas component.

## Description

### TECHNICAL FIELD

The present invention relates to a vasodilator composition, a vasodilator composition kit, a pharmaceutical composition for a disease caused by a vascular stenosis or vascular occlusive disorder, and a pharmaceutical composition kit for a disease caused by a vascular stenosis or vascular occlusive disorder.

### BACKGROUND ART

Nitric oxide (NO), which is a gas, is commonly used as a medicine. Specifically, treatment is performed by allowing a patient with pulmonary hypertension or the like to inhale NO in order to take NO into a pulmonary blood vessel, thereby inducing pulmonary vasodilation (Non-Patent Literature 1).

### Citation List

### Patent Literature

### Non-Patent Literature

Non-Patent Literature 1: "pulmonary vasodilator (inhalation gas) INOflo® for inhalation 800 ppm," October 2017, AIR WATER INC, Mallinckrodt Manufacturing LLC, and Sumitomo Seika Chemicals Company, Limited

### SUMMARY OF INVENTION

### Technical Problem

However, NO easily reacts with oxygen, and when NO reacts with oxygen NO is converted into nitrogen dioxide (NO₂). NO₂ is known to be the most toxic of the nitrogen oxides and causes disorders of respiratory organs such as the lungs. Therefore, if the NO concentration is increased at the time of inhalation into the lungs in an attempt to increase the administration concentration of NO to the patient, a high concentration of NO₂ may occur at the time of inhalation, which may cause respiratory disorders. Therefore, NO has a problem in that it is difficult to control the dosage.

With the foregoing in mind, the object of the present invention is to provide a vasodilator composition capable of adjusting the dosage of NO.

### Solution to the Problem

In order to achieve the above objective, the present invention provides a vasodilator composition (hereinafter also referred to as "composition") including microbubbles, wherein the microbubbles contain NO as a gas component.

The present invention also provides a vasodilator composition kit (hereinafter also referred to as "composition kit"), including a vasodilator composition and another component, wherein the vasodilator composition and the other component are arranged in isolation, and the vasodilator composition is the vasodilator composition according to the present invention.

The present invention also provides a pharmaceutical composition for a disease (hereinafter also referred to as a "pharmaceutical composition") caused by a vascular stenosis or vascular occlusive disorder (hereinafter also referred to as a "vascular stenosis"), including the vasodilator composition according to the present invention.

The present invention also provides a pharmaceutical kit for a disease caused by a vascular stenosis or vascular occlusive disorder (hereinafter also referred to as a "pharmaceutical kit"), including the vasodilator composition kit according to the present invention. Advantageous Effects of the Invention

According to the present invention, the dosage of NO can be adjusted.

### BRIEF DESCRIPTION OF DRAWINGS

[FIG. 1] FIG. 1 is a cross-sectional view showing an example of a double-chamber container in which the composition of the present invention and other component are contained.
[FIG. 2] FIG. 2 is a schematic diagram showing a microbubble-production apparatus in Example 1.
[FIG. 3A] FIG. 3A is a graph showing relative values of intracellular Ca²⁺ concentrations in Example 1.
[FIG. 3B] FIG. 3B is a graph showing relative values of intracellular Ca²⁺ concentrations in Example 1.
[FIG. 3C] FIG. 3C is a graph showing relative values of intracellular Ca²⁺ concentrations in Example 1.
[FIG. 3D] FIG. 3D is a graph showing relative values of intracellular Ca²⁺ concentrations in Example 1.
[FIG. 4] FIGs. 4A and 4B show graphs showing changes in blood pressure over time in Example 2.
[FIG. 5] FIGs. 5A and 5B show graphs showing electrocardiograms in Example 3.
[FIG. 6] FIGs. 6A and 6B show graphs showing results of blood pressure and electrocardiogram in Example 3.
[FIG. 7] FIG. 7 is a graph showing the coronary perfusion rate in Example 4.

### DESCRIPTION OF EMBODIMENTS

### <Vasodilator Composition>

The vasodilator composition of the present invention includes microbubbles, wherein the microbubbles contain nitric oxide (NO) as a gas component. The composition of the present invention is characterized in that it includes these microbubbles, and other configurations and conditions are not particularly limited. Since the composition of the present invention contains NO as a microbubble, it can be directly administered into the body of a subject of administration, for example, by intravenous administration or the like. Therefore, according to the composition of the present invention, by increasing or decreasing the amount of the composition to be administered to the subject of administration, the dosage to the subject of administration can be increased or decreased. Thus, according to the composition of the present invention, it is possible to adjust the dosage of NO, and it is also possible to adjust, for example, the degree of vasodilation. Since the composition of the present invention can be administered directly into the body of the subject of administration, for example, local administration as well as systemic administration can be performed, as in administration by inhalation, for example.

In the present invention, "vasodilation" means dilation of the inner diameter of a blood vessel caused by relaxation of vascular endothelial cells. The blood vessel may be, for example, either an artery or a vein, but is preferably an artery. The vasodilation may be evaluated directly, for example, by measuring the inner diameter of a blood vessel, or indirectly by measuring other indices. The direct evaluation can be made, for example, by measuring the inner diameter of a blood vessel of a subject of administration using an ultrasonic diagnostic apparatus or the like. Examples of the blood vessels of a subject of administration include blood vessels around the site of administration of the composition of the present invention, coronary arteries of the heart, and pulmonary arteries. It is also known that blood pressure decreases when dilation of blood vessels occurs in a subject of administration. Therefore, the indirect evaluation can be made by, for example, using blood pressure as the other indicator and measuring the blood pressure in the subject of administration. The blood pressure is, for example, mean blood pressure. In the present invention, for example, when the inner diameter of a blood vessel of a subject administered with the composition of the present invention is significantly dilated (as compared with the inner diameter of the blood vessel of the subject not administered with the composition of the present invention or as compared to the inner diameter of a blood vessel of a subject administered with the same composition as the target composition except that the microbubbles do not contain NO), it can be evaluated that vasodilation has been achieved.

In the present invention, a "microbubble" means a closed minute space made of a gas surrounded by something other than a gas, and can also be referred to as a microscopic bubble. The microbubbles may be, for example, fine bubbles. The fine bubble (FB) generally means a microbubble having a bubble diameter of less than 100 µm. The bubble diameter means the spherical equivalent diameter of the bubble. The bubble diameter may be a mean diameter (arithmetic mean diameter) of microbubble obtained by the measurement method to be described below. A fine bubble may be a microbubble or an ultrafine bubble (UFB). The microbubbles generally mean bubbles having a bubble diameter of 1 µm or more and less than 100 µm. Ultrafine bubbles generally mean microbubbles having a bubble diameter of less than 1 µm.

The microbubbles are present dispersed in a medium. The microbubbles are present dispersed in whole or in part in the medium. In the latter case, the microbubbles may also be localized to a part of the medium. The medium can be, for example, a liquid or solid. Examples of the liquid include an aqueous solvent including water, an oily solvent, or a mixed solvent thereof. Further, the liquid includes a sol. Examples of the solid include a solid obtained by coagulating the liquid. Further, the solid includes a gel. Examples of the liquid include physiological saline; a buffer such as a phosphate buffer; an infusion such as an extracellular solution or an intracellular solution; water such as distilled water or pure water; a cell-culture medium such as DMEM, RPMI1640; and an organ-storage solution. The solid may be, for example, a solidified matter of the liquid.

The microbubbles may contain only NO or may also contain another gas in a gas form (gas component). The NO can also be regarded as an active component in the microbubbles, for example. Examples of the other gas include carbon monoxide (CO); a biogas such as hydrogen sulfide (H2S), hydrogen (H2), and the like; a rare gas such as helium (He), argon (Ar), krypton (Kr), xenon (Xe), and the like; carbon dioxide (CO₂); nitrous oxide (N₂O); nitrogen (N₂); methane (CH₄); ethane (CH3CH3); propane (CH3CH2CH3); fluoromethane (CH₃F); difluoromethane (CH₂F₂); carbon tetrafluoride (CF₄); ethylene oxide (C₂H₄O); and air. The "biological gas" in the present invention means a gas containing carbon monoxide (CO), nitric oxide (NO), hydrogen sulfide (H2S), hydrogen (H2), or a mixed gas containing two or more of these. In the presence of oxygen or ozone, nitric oxide reacts with oxygen or ozone to form nitrogen dioxide (NO₂). Also, nitrogen dioxide is known to be toxic. Thus, it is preferable that the microbubbles are substantially free of oxygen or ozone, for example. "Substantially free" means, for example, that the concentration of oxygen or ozone in the sample is at a concentration below the detection limit in the gas chromatograph. When the microbubbles contain two or more gas components, it is preferable that the gas component other than NO is a gas component that does not react with NO, such as a rare gas or nitrogen, for example. The microbubbles, for example, exclude the case in which the gas is air only. In the present invention, the "air" means, for example, air (atmosphere) used in producing the microbubbles. When there is a medical gas grade, the gas in the microbubbles is preferably a gas derived from a medical gas.

The microbubble density means the number of microbubbles relative to the volume of the medium. The "density" can also be referred to as a number concentration. The lower limit of the microbubble density is, for example, 5×10⁵ bubbles/ml, 1×10⁶ bubbles/ml, 5×10⁶ bubbles/ml, 1×10⁷ bubbles/ml, 5×10⁷ bubbles/ml, 1×10⁸ bubbles/ml, 5×10⁸ bubbles/ml, 1×10⁹ bubbles/ml, preferably 1×10⁶ bubbles/ml, 5×10⁶ bubbles/ml, 1×10⁷ bubbles/ml, 5×10⁷ bubbles/ml, 1×10⁸ bubbles/ml, or 5×10⁸ bubbles/ml. The upper limit of the microbubble density is, for example, 1.5×10⁹ bubbles/ml, 2×10⁹ bubbles/ml, 3×10⁹ bubbles/ml, 5×10⁹ bubbles/ml, 7×10⁹ bubbles/ml, 9×10⁹ bubbles/ml, 1×10¹⁰ bubbles/ml, 5×10¹⁰ bubbles/ml, 1×10¹¹ bubbles/ml, 5×10¹¹ bubbles/ml, 1×10¹² bubbles/ml, or 5×10¹² bubbles/ml. The microbubble density is in the range, for example, from 5×10⁵ bubbles/ml to 5×10¹² bubbles/ml, 5×10⁵ bubbles/ml to 1×10¹² bubbles/ml, 5×10⁵ bubbles/ml to 5×10¹¹ bubbles/ml, 5×10⁵ bubbles/ml to 1×10¹¹ bubbles/ml, 5×10⁵ bubbles/ml to 5×10¹⁰ bubbles/ml, 5×10⁵ bubbles/ml to 1×10¹⁰ bubbles/ml, 1×10⁶ bubbles/ml to 9×10⁹ bubbles/ml, 5×10⁶ bubbles/ml to 9×10⁹ bubbles/ml, 1×10⁷ bubbles/ml to 7×10⁹ bubbles/ml, 5×10⁷ bubbles/ml to 7×10⁹ bubbles/ml, 1×10⁸ bubbles/ml to 5×10⁹ bubbles/ml, 5×10⁸ bubbles/ml to 5×10⁹ bubbles/ml, 1×10⁹ bubbles/ml to 3×10⁹ bubbles/ml, 5×10⁸ bubbles/ml to 2×10⁹ bubbles/ml, or 5×10⁸ bubbles/ml to 1.5×10⁹ bubbles/ml.

The density, bubble diameter, and mean diameter (hereinafter also referred to as "characteristics") of the microbubbles can be appropriately measured according to the medium in which the microbubbles are dispersed. When the microbubbles are dispersed in a liquid medium, the characteristics of the microbubbles can be calculated by analyzing the bubbles in the composition of the present invention by a particle-trajectory-analysis method. The particle-trajectory-analysis method can be performed using, for example, NanoSight^{®} NS300 (manufactured by Malvern Instruments) in accordance with Example 1, to be described below. The characteristics of the microbubbles may be calculated by an analysis method other than the particle-trajectory-analysis method. In this case, the characteristics of the microbubbles obtained by the other analysis method satisfy the above-mentioned exemplification when converted into the calculation value obtained by the particle-trajectory-analysis method. When the microbubbles are dispersed in a solid medium, the characteristics of the microbubbles can be calculated based on the characteristics of the microbubbles in the liquid before solidification of the medium and the characteristics of the microbubbles in the liquid obtained by dissolving the solid medium.

The proportion of NO in the gas is, for example, greater than 0%, 100% or less, 10% to 100%, 20% to 100%, 30% to 100%, 40% to 100%, 50% to 100%, 60% to 100%, 70% to 100%, 80% to 100%, 90% to 100%, 95% to 100%, 96% to 100%, 97% to 100%, 98% to 100%, or 99% to 100%, and preferably 90% to 100%.

The composition of the present invention can be produced, for example, by a method for producing microbubbles such as fine bubbles using any gas. Thus, a method for producing the composition of the present invention includes, for example, the step of producing microbubbles using a gas containing NO and a medium. As a specific example, when the composition of the present invention is a liquid, the liquid composition can be produced by using, for example, a gas containing NO, the medium, and a microbubble-production apparatus of a swirling flow type, an ejector type, a venturi type, a static mixer type, a micropore type, a pressure dissolution type, or an ultrasonic cavitation type. In addition, when the composition of the present invention is a solid, the solid composition can be produced by coagulating the liquid composition by a known method. When the solid is a gel, the gel composition can be produced, for example, by mixing the liquid composition with a gelling agent. At the start of the producing microbubbles, the state of the gas containing NO may be a gas, a liquid, or a solid. The gas containing NO may include a plurality of types of gases. In this case, each gas may be separately subjected to the producing microbubbles, or all or some of the gases containing NO may be subjected to the producing microbubbles at the same time. As a specific example, when the gas is NO and CO, NO and CO may be introduced simultaneously or separately.

The composition of the present invention may be used, for example, in vivo or in vitro. The composition of the present invention can also be used, for example, as a reagent for research, and can be used as a pharmaceutical. In the latter case, the composition of the present invention may also be referred to as a medicament or pharmaceutical composition for a disease caused by vascular stenosis.

The subject of administration of the composition of the present invention is not particularly limited. When the composition of the present invention is used in vivo, examples of the subject of administration include humans and nonhuman animals. Examples of nonhuman animals include mammals such as mice, rats, rabbits, dogs, sheep, horses, cats, goats, monkeys, guinea pigs, and the like, in addition to birds and fish. When the composition of the present invention is used in vitro, examples of the subject of administration include cells, tissues, and organs. Examples of cells include cells collected from a living body and cultured cells. Examples of tissues and organs include tissues (biological tissues) or organs collected from a living body. Examples of cells include vascular endothelial cells and vascular smooth muscle cells.

The use condition (administration condition) of the composition of the present invention is not particularly limited, and, for example, an administration method, an administration period, a dosage, and the like can be appropriately determined depending on the type of the subject of administration, and the like.

The dosage of the composition of the present invention is not particularly limited. When the composition of the present invention is used in vivo, the dosage can be appropriately determined, for example, depending on the type, symptom, age, administration method, and the like, of the subject of administration. As a specific example, in the case of administering a composition having a microbubble density between 1×10⁸ bubbles/ml and 5×10¹² bubbles/ml to humans, the dosage of NO per day is, for example, 0.1 to 10 ml/kg body weight. The number of administrations per day of the composition of the present invention is, for example, 1 to 5 times or 1 to 3 times, and is preferably 1 time. In the case of intravenous administration to an adult human, the dosage of NO per day is, for example, 2.5 mg, and the number of administrations per day is, for example, 1 time. In the composition of the present invention, the content of the compound is not particularly limited, and can be appropriately set according to, for example, the aforementioned dosage per day. The composition of the present invention may be administered continuously or discontinuously, for example. The discontinuous administration may be, for example, intermittent administration. The composition of the present invention may be administered, for example, at predetermined intervals. The predetermined intervals may be substantially equal intervals, equal intervals, or unequal intervals. The predetermined interval may be, for example, an 8-to-12-hour interval, a one-day interval, or the like.

The administration method of the composition of the present invention is not particularly limited. When the composition of the present invention is administered in vivo, it may be administered orally or parenterally. Examples of the parenteral administration include intravenous injection (intravenous administration), intramuscular injection (intramuscular administration), transdermal administration, subcutaneous administration, intradermal administration, enteral administration, rectal administration, vaginal administration, nasal administration, pulmonary administration, intraperitoneal administration, and topical administration.

The dosage form of the composition of the present invention is not particularly limited, and can be appropriately determined depending on, for example, the administration method. Examples of the dosage form include a liquid form and a solid form. Specific examples of the dosage form include preparations for oral administration such as controlled-release formulations (enteric formulations, sustained release formulations, etc.), capsules, liquids and solutions for oral administration (elixirs, suspensions, emulsions, aromatic waters, lemonade, etc.), syrups (preparation for syrups, etc.), granules (effervescent granules, fine granules, etc.), powders, tablets (orally disintegrating tablets/orodispersible tablets, chewable tablets, effervescent tablets, dispersible tablets, soluble tablets, coated tablets, etc.), pills, jellies for oral administration, and the like; preparations for oro-mucosal application such as tablets for oro-mucosal application (medicated chewing gums, sublingual tablets, troches/lozenges, drops, buccal tablets, mucoadhesive tablets, etc.), sprays for oro-mucosal application; semisolid preparations for oro-mucosal application, preparations for gargles, and the like; preparations for injection such as injections (implants/pellets, prolonged-release injections, parenteral infusions (preparations for infusion), lyophilized injections, powder for injections, prefilled syringes, cartridge, etc.); preparations for dialysis such as dialysis agents (peritoneal dialysis agents and hemodialysis agents), and the like; preparations for inhalation such as inhalations (metered-dose inhalers, inhalation solutions, dry-powder inhalers, etc.); preparations for ophthalmic application such as ophthalmic ointments, ophthalmic preparations, and the like; preparations for otic application such as ear preparations; preparations for nasal application such as nasal preparations (nasal solutions, nasal dry-powder inhalers, etc.) and the like; preparations for rectal application such as suppositories for rectal application, semi-solid preparations for rectal application, enemas for rectal application, and the like; preparations for vaginal application such as suppositories for vaginal use, tablets for vaginal use, and the like; and preparations for cutaneous application such as liquids and solutions for cutaneous application (spirits, liniments, lotions, etc.), creams, gels, solid dosage forms for cutaneous application (powders for cutaneous application, etc.), sprays for cutaneous application (aerosol for cutaneous application, pump sprays for cutaneous application, etc.), patches (tapes/plasters, cataplasms/gel patches, etc.), ointments, and the like. When the composition of the present invention is administered orally, examples of the dosage form include tablets, coated tablets, pills, fine granules, granules, powders, capsules, solutions, syrups, emulsions, and suspensions. When the composition of the present invention is administered parenterally, examples of the dosage form include preparations for injection and preparations for infusion. When the composition of the present invention is administered transdermally, examples of the dosage form include topical agents such as patches, embrocations, ointments, creams, and lotions.

The composition of the present invention may include, for example, an additive if necessary, and when the composition of the present invention is used as a pharmaceutical or a pharmaceutical composition, it is preferred that the additive be a pharmaceutically acceptable additive or include a pharmaceutically acceptable carrier. The additive is not particularly limited, and examples thereof include an osmotic regulator such as a salt, a base raw material, an excipient, a colorant, a lubricant, a binder, a disintegrant, a stabilizer, a coating agent, a preservative, a pH-adjusting agent, and a flavoring agent such as a perfume. In the present invention, the amount of the additive to be blended is not particularly limited as long as it does not hinder the function of NO.

Examples of the excipient include sugar derivatives such as lactose, lactose hydrate, sucrose, glucose, mannitol, sorbitol, and the like; starch derivatives such as corn starch, potato starch, α starch, dextrin, and the like; cellulose derivatives such as crystalline cellulose; gum arabic; dextran; organic excipients such as pullulan and the like; silicate derivatives such as light anhydrous silicic acid, synthetic aluminum silicate, calcium silicate, magnesium metasilicate, and the like; phosphates such as calcium hydrogen phosphate, and the like; carbonates such as calcium carbonate, and the like; and inorganic excipients such as sulfates such as calcium sulfate. The colorant may be, for example, yellow ferric oxide. Examples of the lubricant include stearic acid metal salts such as stearic acid, calcium stearate, magnesium stearate, and the like; talc; polyethylene glycol; silica; and hydrogenated vegetable oil. Examples of the flavoring agent include perfumes such as cocoa powder, menthol, aromatic powder, mint oil, borneol, cinnamon powder, and the like; sweeteners; and acidulants. Examples of the binder include hydroxypropylcellulose, hydroxypropylmethylcellulose, polyvinylpyrrolidone, and macrogol. Examples of the disintegrant include cellulose derivatives such as carboxymethylcellulose, calcium carboxymethylcellulose, and the like; chemically modified starches such as carboxymethylstarch, sodium carboxymethylstarch, cross-linked polyvinylpyrrolidone, sodium starch glycolate, and the like; and chemically modified celluloses. Examples of the stabilizer include paraoxybenzoic acid esters such as methyl paraben, propylparaben, and the like; alcohols such as chlorobutanol, benzyl alcohol, phenylethyl alcohol, and the like; benzalkonium chloride; phenols such as phenol, cresol, and the like; thimerosal; dehydroacetic acid; and sorbic acid. Examples of the coating agent include hypromellose and macrogols such as macrogol 6000, talc, and titanium oxide.

The composition of the present invention can dilate a blood vessel of a subject of administration, for example. Thus, the composition of the present invention can be suitably used, for example, as a therapeutic agent for a disease caused by vascular stenosis.

### <Vasodilator Composition Kit>

As described above, the vasodilator composition kit of the present invention includes a vasodilator composition and another component, wherein the vasodilator composition and the other component are arranged in isolation, and the vasodilator composition is the vasodilator composition according to the present invention. The composition kit of the present invention is characterized in that it includes the vasodilator composition, wherein the vasodilator composition is the vasodilator composition according to the present invention, and other configurations and conditions are not particularly limited. According to the composition kit of the present invention, by adjusting the dosage of the composition, the dosage of NO can be adjusted. Regarding the composition kit of the present invention, reference can be made to the description as to the composition of the present invention.

The other component is not particularly limited and may be appropriately determined depending on the contents of the composition and the purpose of administration to the subject of administration, and examples thereof include the aforementioned additives, drugs, and nutritional agents. The drug may be, for example, an antibiotic. When the osmotic pressure of the composition is not adjusted, it is preferable that the other component contain an osmotic pressure regulator (regulating substance). Examples of the osmotic pressure regulating substance include sugars such as glucose; salts (electrolytes) such as sodium chloride, calcium chloride, calcium chloride, sodium bicarbonate, magnesium chloride, and the like; amino acids; and proteins. Examples of the nutritional agent include sugars such as glucose, and the like; and vitamins. The other component may be a solid or liquid. In the former case, the other component is preferably arranged in an undissolved state in a solvent or the like, and the other component is preferably configured to be dissolved, for example, when mixed with the composition. In the latter case, the other component is preferably dissolved in, for example, a solvent.

In the composition kit of the present invention, the composition and the other component are arranged in isolation, that is, the composition and the other component are arranged in an unmixed state or not in contact with each other. Specifically, the composition and the other component are disposed at different locations in a container containing them.

In the composition kit of the present invention, it is preferable that the composition and the other component be contained in a container. In this case, the container includes a first chamber for containing the composition and a second chamber for containing the other component. In the container, the first chamber and the second chamber may be configured independently, that is, may be configured as independent containers, or may be configured integrally, that is, may be configured as a single container. When the first chamber and the second chamber are configured as a single container, the container preferably includes an isolation portion capable of isolating the first chamber and the second chamber. When the container includes the isolation portion, the first chamber and the second chamber are arranged with the isolation portion interposed therebetween, for example. When the composition and the other component are mixed and administered to a subject of administration, the isolation portion is preferably configured to be capable of communicating the first chamber with the second chamber.

As the container, including the first chamber and the second chamber, for example, a double-chamber container for medical use can be used. As the double-chamber container, for example, a plastic double bag (e.g., JP 2016-190646 A, JP 2016-131577 A) in which a plurality of chambers are formed by providing the isolation portion in the plastic bag, a dissolution liquid kit (e.g., WO 96/25136) in which a container containing the other component and a container containing a dissolution liquid (corresponding to the composition) are integrated in a communicable manner, a double-chamber type prefilled syringe (e.g., JP 2012-245086 A) and the like can be used.

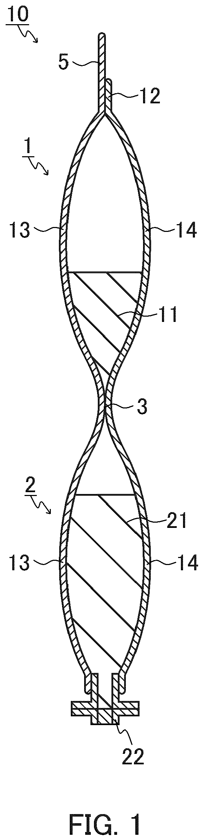

In the composition kit of the present invention, an example of the double-chamber container in which the composition and the other component are contained will be described with reference to FIG. 1. FIG. 1 is a cross-sectional view showing an example of the composition kit of the present invention. As shown in FIG. 1, the composition kit includes a container 10, a composition 11, and other component 21. The container 10 includes a first chamber 1 containing the composition 11, a second chamber 2 containing the other component 21, and an isolation portion 3 that isolates the first chamber 1 and the second chamber 2 and allows the first chamber 1 and the second chamber 2 to communicate with each other. The container 10 further includes a hanging portion 5 capable of hanging the container 10.

As shown in FIG. 1, the container 10 is formed of a sheet 13, a sheet 14, and a discharge portion (discharge port) 22. As to the sheets 13 and 14, as shown in FIG. 1, the upper-end portions of the sheet 14 and the sheet 13 are welded to form an upper-end portion 12 of the first chamber 1, and lower-end portions of the sheets 13 and 14 are connected to the discharge portion 22. Further, the sheets 13 and 14 are welded at the central portion thereof to form the isolation portion 3. The welding of the isolation portion 3 is peelable, and the welding of sheets 13 and 14 at the isolation portion 3 is released by applying pressure to the first chamber 1, so that the first chamber 1 and the second chamber 2 can communicate with each other. In the container 10, the first chamber 1 is a space from the upper-end portion 12 to the isolation portion 3 inside sheets 13 and 14. In the container 10, the second chamber 2 is a space from the separation portion 3 to the discharge portion 22 inside the sheets 13 and 14.

Plastic sheets can be used as the sheets 13 and 14. The plastic sheet preferably includes a plurality of layers, for example, an inner surface layer, an outer surface layer, and an intermediate layer. As the inner surface layer and the outer surface layer, for example, a thermoplastic resin such as a thermoplastic olefin-based resin, a thermoplastic propylene-based resin, or a thermoplastic polyethylene-based resin can be used. When such a thermoplastic resin is used, by laminating the sheets 13 and 14 in such a manner to face each other, and performing heat sealing, the outer peripheral portions of the first chamber 1 and the second chamber 2, the upper-end portion 12, and the isolation portion 3 can be easily formed, thereby producing the container 10. For example, a resin having high flexibility is preferred for the intermediate layer, and as a specific example, a thermoplastic olefin-based resin composition can be used.

The volume and shape of the first chamber 1 and the second chamber 2 are not particularly limited, and can be appropriately set depending on the dosage of the composition and other component, for example.

The composition kit of the present invention is capable of dilating, for example, a blood vessel of a subject of administration. Thus, the composition kit of the present invention can be suitably used, for example, as a therapeutic agent for a disease caused by vascular stenosis.

### <Pharmaceutical Composition>

A pharmaceutical composition for a disease caused by a vascular stenosis or vascular occlusive disorder of the present invention includes the vasodilator composition of the present invention. The pharmaceutical composition of the present invention is characterized in that it includes the composition of the present invention, and other configurations and conditions are not particularly limited. According to the pharmaceutical composition of the present invention, by adjusting the dosage of the composition, the dosage of NO can be adjusted. According to the pharmaceutical composition of the present invention, the stenosed blood vessel can be dilated so that a disease caused by a vascular stenosis or vascular occlusive disorder can be treated. Regarding the pharmaceutical composition of the present invention, reference can be made to the descriptions as to the composition and composition kit of the present invention.

In the present invention, the "vascular stenosis or vascular occlusive disorder" means, for example, an increase in vascular resistance or poor circulation which causes a vascular insufficiency.

Examples of the disease caused by the vascular stenosis include angina pectoris, myocardial infarction, cerebral infarction, transient cerebral ischemic attack, pulmonary hypertension, and acute heart failure. The pharmaceutical composition of the present invention can also be used for the treatment of attack of angina (anginal attack).

In the present invention, the term "treatment" may be used in any sense of the treatment, prevention, improvement, remission, amelioration, suppression of symptom development, and/or cessation of symptom development. Thus, the pharmaceutical composition of the present invention may also be referred to as, for example, a treatment agent, a prevention agent, an improvement agent, a remission agent, an amelioration agent, a development suppression agent, and/or a development cessation agent.

The pharmaceutical composition of the present invention may be used as a pharmaceutical composition for a disease other than the disease caused by vascular stenosis.

### <Pharmaceutical Kit>

A pharmaceutical kit for a disease caused by a vascular stenosis or vascular occlusive disorder of the present invention (hereinafter also referred to as a "pharmaceutical kit") includes the vasodilator composition kit of the present invention. The pharmaceutical kit of the present invention is characterized in that it includes the composition kit of the present invention, and other configurations and conditions are not particularly limited. According to the pharmaceutical kit of the present invention, by adjusting the dosage of the composition, the dosage of NO can be adjusted. According to the pharmaceutical kit of the present invention, since the stenosed blood vessel can be dilated, a disease caused by a vascular stenosis or vascular occlusive disorder can be treated. Regarding the pharmaceutical kit of the present invention, reference can be made to the descriptions as to the composition, composition kit, and pharmaceutical composition of the present invention.

The pharmaceutical kit of the present invention may be used as a pharmaceutical kit for a disease other than the disease caused by a vascular stenosis or vascular occlusive disorder.

### <Treatment>

A method for treating a disease caused by a vascular stenosis or vascular occlusive disorder of the present invention (hereinafter also referred to as a "treatment method") includes the step of administering to a patient the vasodilator composition of the present invention. The treatment method of the present invention is characterized in that it administers the composition of the present invention, and other steps and conditions are not particularly limited. According to the treatment method of the present invention, by adjusting the dosage of the composition, the dosage of NO can be adjusted. According to the treatment method of the present invention, since the stenosed blood vessel can be dilated, a disease caused by a vascular stenosis or vascular occlusive disorder can be treated. Regarding the treatment method of the present invention, reference can be made to the descriptions as to the composition, composition kit, pharmaceutical composition, and pharmaceutical kit of the present invention.

The treatment method of the present invention can also be referred to as a therapy performed on a patient with a disease caused by a vascular stenosis or vascular occlusive disorder, for example. Thus, the treatment method of the present invention can also be referred to as a therapy method for a disease caused by a vascular stenosis or vascular occlusive disorder, for example.

The treatment method of the present invention may use the pharmaceutical composition as the composition. In addition, in the treatment method of the present invention, the composition kit or the pharmaceutical kit (which hereinafter may be collectively referred to as the "kit") may be used as the composition.

When the treatment method of the present invention uses the kit, in the administering, the composition and the other component may be administered simultaneously or separately. When the composition and the other component are administered simultaneously, it is preferable that the treatment method of the present invention include the step of mixing the composition and the other component in the kit prior to the administering. In this case, in the administering, the resulting mixture is administered to a patient.

Regarding the conditions of administration in the administering, reference can be made to the above description.

### Examples

Next, examples of the present invention will be described. The present invention, however, is not limited by the following examples.

### Example 1

It was examined that the composition of the present invention lowers calcium ion concentration in cardiomyoblasts.

NO lowers the calcium ion concentration in vascular smooth muscle cells, and therefore inhibits the contraction of vascular endothelial cells, thereby achieving vasodilation. Therefore, it was examined that the composition of the present invention has a vasodilator function by examining whether the intracellular calcium ion (Ca²⁺) concentration is lowered in cardiomyoblasts by the composition of the present invention.

### (1) Production of Composition

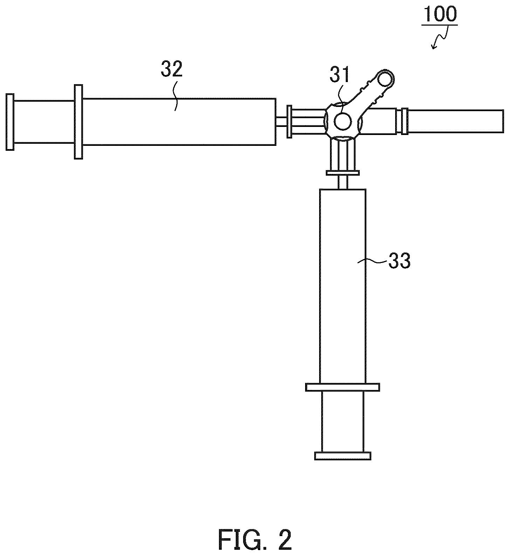

The composition of the present invention was produced using a microbubble-production apparatus 100 shown in FIG. 2. As shown in FIG. 2, the production apparatus 100 includes a three-way stopcock 31 and syringes 32 and 33 disposed on two sides of the three-way stopcock 31. In the production apparatus 100, the syringes 32 and 33 communicate with each other through the three-way stopcock 31. First, the syringe 32 was released from the three-way stopcock 31, and 20 ml of Earle's balanced salt solution (EBSS) was introduced into the inside of the syringe 32. The composition of EBSS was as follows: 26 mmol/l NaHCO₃, 1 mmol/l NaH₂PO₄, 5.4 mmol/l KCl, 116 mmol/l NaCl, 5.5 mmol/l glucose, and 2 mmol/l CaCl₂, and pH 7 4. Next, the syringe 32 was again connected to the three-way stopcock 31 and the gas in the three-way stopcock 31 was removed. After the removal, the syringe 33 was released from the three-way stopcock 31, and 20 ml of medical nitric oxide (NO concentration: 99.0 (v/v)% or more, manufactured by TAIYO NIPPON SANSO CORPORATION) was introduced into the inside of the syringe 33. Then the syringe 33 was again connected to the three-way stopcock 31. After the connection, the plunger of each of the syringes 32 and 33 was continuously pistoned in an outer cylinder for 10 minutes to produce microbubbles containing NO as a gas component, thereby producing the composition of the present invention (the composition of Example 1-1). Further, the composition (the composition of Examples 1-2) was produced in the same manner except that the dissolved air in EBSS was removed by degassing with argon (Ar-degassing) in advance.

### (2) Characteristics of Composition

With respect to the composition obtained by Example 1-1 and the composition obtained by diluting these compositions 10 times or 100 times, the composition was left standing for about 1 hour, and then the physical properties of the composition were measured with a default parameter using NanoSight^{®} NS300 (manufactured by Malvern Instruments). Incidentally, the measurement was performed at 25°C. As a result, the microbubble mean diameter and the microbubble density in the composition were as follows.
(Composition of Example 1-1 (NO UFB))
   No dilution (×1):
      Mean diameter: 116.1 ± 39.8 nm, density: 3.52×10⁹ ± 1.12×10⁸ bubbles/ml;
   10-fold dilution (×10):
      Mean diameter: 116.1 ± 39.8 nm, density: 1.06×10⁹ ± 2.97×10⁷ bubbles/ml;
   100-fold dilution (×100):
      Mean diameter: 113.4 ± 39.2 nm, density: 1.51×10⁸ ± 6.57×10⁶ bubbles/ml;
(Composition of Example 1-2 (Ar-substituted, Ar NO UFB))
   No dilution (×1):
      Mean diameter: 137.0 ± 48.2 nm, density: 2.89×10⁹ ± 4.10×10⁷ bubbles/ml;
   10-fold dilution (×10):
      Mean diameter: 126.3 ± 49.5 nm, density: 7.81×10⁸ ± 1.38×10⁷ bubbles/ml;
   100-fold dilution (×100):
      Mean size: 116.4 ± 43.0 nm, Density: 1.13×10⁸ ± 3.29×10⁶ bubbles/ml

### (3) Measurement of calcium ion concentration

Rat cardiomyoblasts (H9C2) were used to measure changes in calcium ion concentrations. The H9C2 cell medium was prepared using DMEM (Dulbecco's Modified Eagle Medium: DMEM ((+) 4.5 g/l D-glucose, (+) 110mg/l pyruvate (-) L-glutamine (manufactured by Gibco)), 10% fetal bovine serum (FBS, manufactured by Biowest Co., Ltd.), 100 mg/l pyruvate (manufactured by Sigma Chemical Co.), 10 ml/l L-glutamine (200 mmol/l 100 × L-glutamine, (manufactured by Gibco)), 100 U/ml penicillin (manufactured by FUJIFILM Wako Pure Chemical Corporation), and 100 µg/ml streptomycin (manufactured by FUJIFILM Wako Pure Chemical Corporation). The H9C2 cells were previously seeded in 96-well plates at 3.0×10³ cells/100 µl per well and cultured until confluent. For the measurement of intracellular Ca²⁺ concentration, Fura 2-AM (Mw.: 1001.85, Cat. No.: F015, manufactured by Dojindo Laboratories), which is a fluorescent probe for measuring [Ca²⁺], was used.

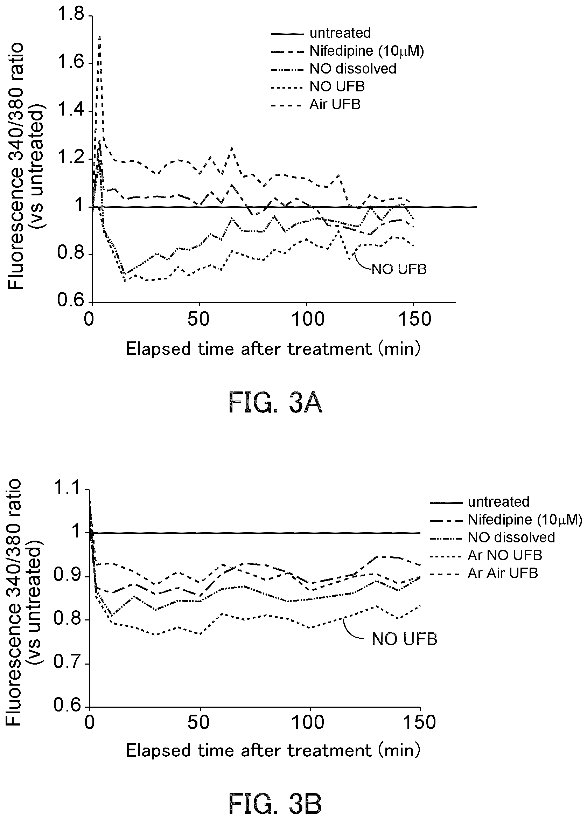

First, a loading buffer was prepared by adding a 1 mmol/l Fura 2-AM DMSO solution to EBSS so as to achieve 5 µmol/l, and further adding Pluronic^{®} F-127 so as to achieve 0.01 (w/v)%, followed by ultrasonic dissolution. Next, after removing the H9C2 cell medium, Fura2-AM was taken into H9C2 cells by adding equal amounts of loading buffer and incubating at 37°C for 20 minutes. After the incubation, the resultant was washed four times with EBSS. Then, after 100 µl of the respective compositions were added and exposed, changes in the fluorescent intensities were measured over time to observe changes in the relative values of the intracellular Ca²⁺ concentration. In the measurement, the wavelength of the excitation light was set at 340 nm or 380 nm, and the measurement wavelength of the resulting fluorescence was set at 510 nm. The relative value of the intracellular Ca²⁺ concentration was calculated as the ratio of the fluorescence intensity at the wavelength of 510 nm with the excitation light having a wavelength of 340 nm (340 nm) to the fluorescence intensity at the wavelength of 510 nm with the excitation light having a wavelength of 380 nm (380 nm) (340 nm/380 nm). The calcium ion concentration of Control 1 (untreated) was measured in the same manner as described above, except that it was untreated, the calcium ion concentration of Control 2 (Air UFB) was measured in the same manner as described above except that a composition produced using air instead of NO was used, and the calcium ion concentration of Control 3 (Nifedipine) was measured in the same manner as described above, except that a 10 µmol/l nifedipine solution was used instead of the composition, and the calcium ion concentration of Control 4 (NO dissolved) was measured in the same manner as described above, except that NO-dissolved EBSS was used instead of the composition. The results are shown in FIGs. 3A to 3D.

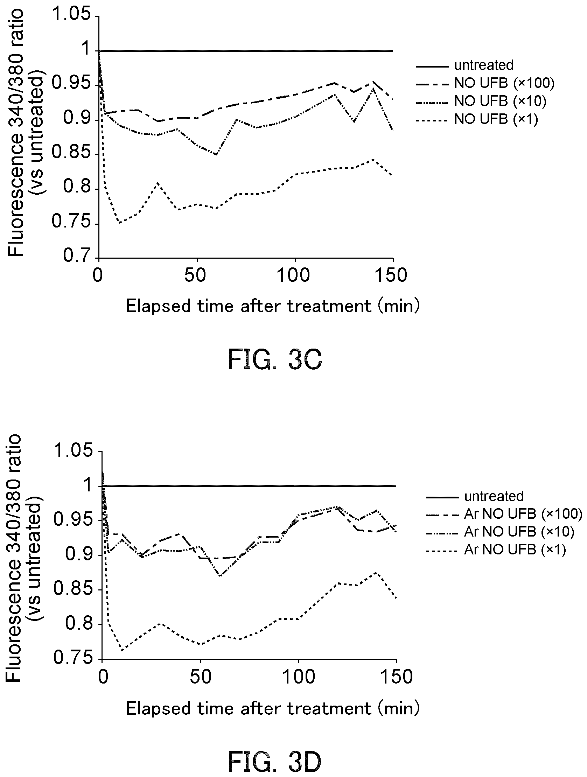

FIGs. 3A to 3D are graphs showing the relative values of intracellular Ca²⁺ concentration. FIG. 3A shows the results of the composition of Example 1-1 of no dilution (× 1) and Controls 1 to 4; FIG. 3B shows the results of the composition of Example 1-1 of no dilution (× 1) using Ar-degassed EBSS and Controls 1 to 5; FIG. 3C shows the results of the dilution series of the composition of Example 1-1 and Control 1; and FIG. 3D shows the results of the dilution series of the composition of Example 1-2 and Control 1. In FIGs. 3A to 3D, the horizontal axis indicates the elapsed time after treatment of the compositions, and the vertical axis indicates the relative value of intracellular Ca²⁺ concentration.

As shown in FIGs. 3A and 3B, the intracellular Ca²⁺ concentrations of Controls 1 to 4 remained almost unchanged or slightly decreased. In contrast, the intracellular Ca²⁺ concentrations of the compositions of Examples 1-1 and 1-2 of no dilution (×1) were significantly decreased. It was also found that the composition of the present invention has a strong effect of decreasing intracellular Ca²⁺ concentrations compared to NO-dissolved solutions.

Next, as shown in FIGs. 3C and 3D, in the compositions of Examples 1-1 and 1-2 of no dilution (× 1), 10-fold dilution (× 10), and 100-fold dilution (× 100), the intracellular Ca²⁺ concentration was decreased in a microbubble density (concentration) dependent manner. When the results of the compositions of Examples 1-1 and 1-2 were compared, no significant differences in the degree of reduction in intracellular Ca²⁺ concentration were observed therebetween, and it was found that NO in the composition of the present invention was hardly oxidized in the composition. From these results it was found that, by adjusting the microbubble density and the microbubble dosage in the composition of the present invention, the degree of its effect can be adjusted, and NO in the microbubbles can be kept stable.

This suggested that the composition of the present invention caused a decrease in calcium ion concentration in cardiomyoblasts and had a vasodilator function.

### Example 2

It was examined that the composition of the present invention has a vasodilating effect.

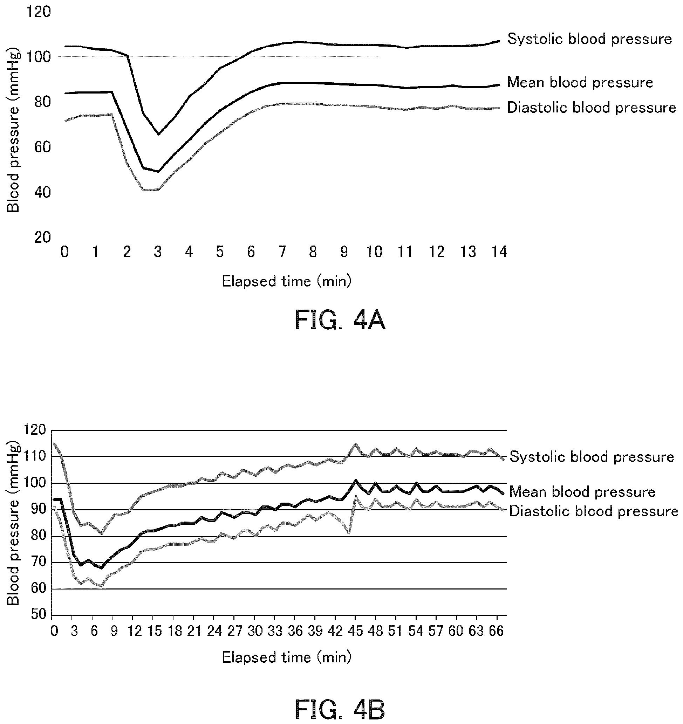

An arterial pressure-sensing probe was inserted from the femoral artery of an anesthetized inbred Lewis rat (♂, 10 weeks old) or a dog (beagle, one-year old, ♂). Then the composition of Example 1-1 of no dilution (× 1) was administered from the femoral vein so as to achieve 1 ml/kg body weight. Blood pressures of the rat and the dog were measured over time after the administration. The results are shown in FIG. 4.

FIG. 4 shows graphs showing changes in blood pressure over time. In FIG. 4A shows the results of the rat and FIG. 4B shows the results of the dog. In FIG. 4, the horizontal axis indicates the time after composition administration and the vertical axis indicates blood pressure. As shown in FIG. 4A and 4B, after the composition administration of Example 1-1, the blood pressure rapidly decreased, and a decrease in blood pressure was sustained up to about 45 minutes after administration.

This showed that the composition of the present invention has a vasodilating effect.

### Example 3

It was examined that the composition of the present invention can suppress angina pectoris.

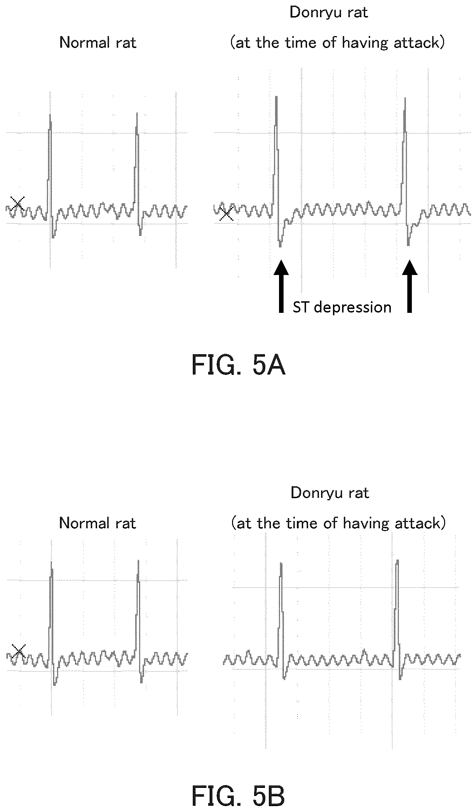

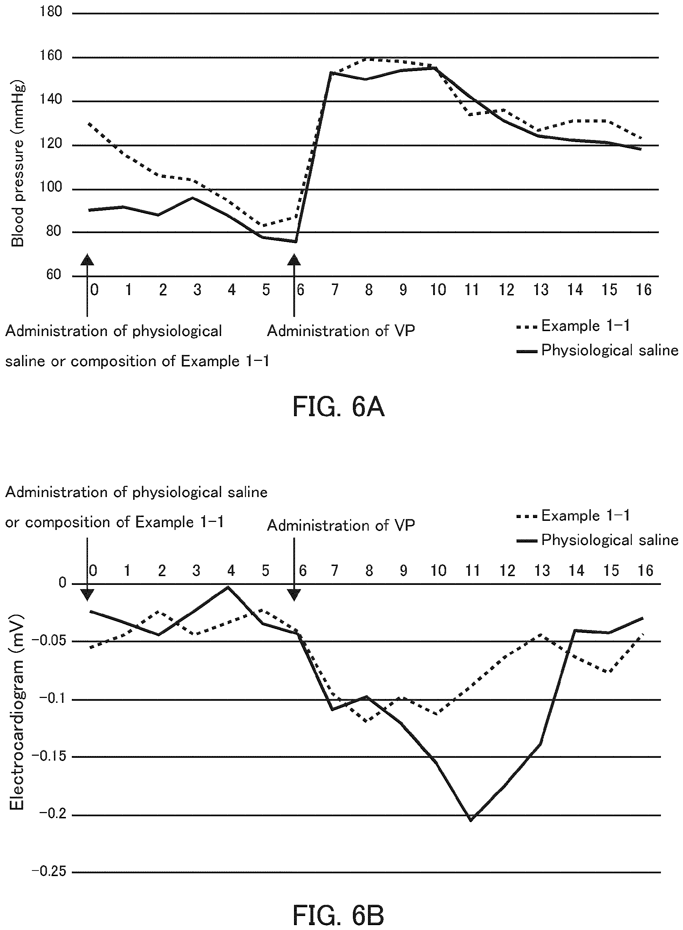

As an angina pectoris model rat, a Donryu rat capable of having an angina pectoris attack by administration of vasopressin (VP) was used. First, an electrocardiograph was attached to the Donryu rat, an arterial pressure-sensing probe was inserted from the femoral artery, and the electrocardiogram and blood pressure were measured over time. The Donryu rat was then administered with 1 ml/kg body weight of physiological saline via the femoral vein. Five minutes after the administration, vasopressin was administered via the femoral vein to induce an anginal attack. The completion of the anginal attack was checked by the electrocardiogram, and then the Donryu rat was administered with the composition of Example 1-1 of 1ml/kg body weight (×1) from the femoral vein. Five minutes after the administration, vasopressin was administered from the femoral vein to induce an anginal attack. The results are shown in FIGs. 5 and 6.

FIG. 5 shows graphs showing an electrocardiogram. In FIG. 5, (A) shows the results of the Donryu rat administered with physiological saline, and (B) shows the results of the Donryu rat administered with the composition of Example 1-1. In FIG. 5, the left graph shows the electrocardiogram of the normal rat, and the right graph shows the electrocardiogram of the rat at the time of having anginal attack by vasopressin. As shown in (A) of FIG. 5, when physiological saline was administered, administration of vasopressin resulted in ST depression characteristic of the angina attack. On the other hand, as shown in (B) of FIG. 5, when the composition of Example 1-1 was administered, the ST depression characteristic of the angina attack was not observed, and the angina attack was suppressed.

Next, FIG. 6 shows graphs showing the results of blood pressure and electrocardiogram. In FIG. 6, (A) shows the results of the blood pressure, and (B) shows the results of the S wave of the electrocardiogram. In (A) of FIG. 6, the horizontal axis indicates the time after administration of physiological saline or the composition of Example 1-1, and the vertical axis indicates the mean blood pressure. In (B) of FIG. 6, the horizontal axis indicates the time after administration of physiological saline or the composition of Example 1-1, and the vertical axis indicates the electrocardiogram. As shown in (A) of FIG. 6, blood pressure decreased after administration of the composition of Example 1-1, and blood vessels were found to be dilated. After the administration of vasopressin, there was no difference between the physiological saline-administration group and the composition of Example 1-1-administration group. On the other hand, as shown in (B) of FIG. 6, after the administration of vasopressin, the S wave was significantly decreased in the physiological saline-administration group, whereas the S wave was suppressed in the composition of Example 1-1-administration group, which showed that the angina attack was suppressed.

This showed that the composition of the present invention can suppress angina pectoris.

### Example 4

It was examined that the composition of the present invention has a vasodilating effect.

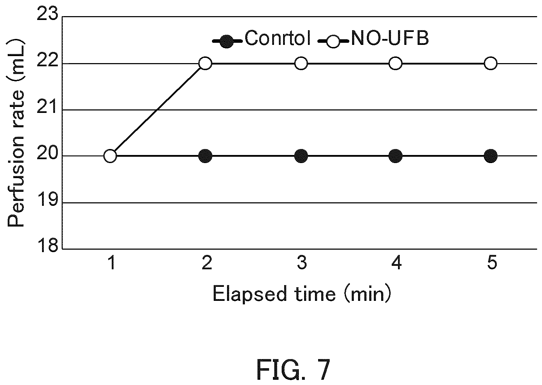

The heart was removed from the Donryu rat of Example 3. Next, the heart was installed in a Langendorff perfusion device and placed in an extracorporeal circulation. In this state, the coronary perfusion rate upon administration of 1 ml of composition of Example 1-1 of no dilution (× 1) to the circulating fluid was measured every 1 minute for 5 minutes. As a control, the measurement was performed in the same manner, except that physiological saline was administered instead of the composition of Example 1-1. The results are shown in FIG. 7.

FIG. 7 is a graph showing coronary artery perfusion. In FIG. 7, the horizontal axis indicates the time after administration of physiological saline or the composition of Example 1-1, and the vertical axis indicates the coronary perfusion rate. As shown in FIG. 7, in the composition of Example 1-1-administration group (NO UFB), the coronary perfusion was increased as compared to the physiological saline-administration group (Control). This is presumed to be because NO in the composition of Example 1-1 allowed the coronary artery to dilate and allowed more fluid volume.

This showed that the composition of the present invention has a vasodilating effect.

While the present invention has been described above with reference to illustrative embodiments and examples, the present invention is by no means limited thereto. Various changes and variations that may become apparent to those skilled in the art may be made in the configuration and specifics of the present invention without departing from the scope of the present invention.

This application claims priority to Japanese Patent Application No. 2020-021195, filed on February 12, 2020, the entire disclosure of which is incorporated herein by reference.

### (Supplementary Notes)

Some or all of the above example embodiments and examples may be described as in the following Supplementary Notes, but are not limited thereto.

### (Supplementary Note 1)

A vasodilator composition, including:
microbubbles, wherein
the microbubbles contain nitric oxide as a gas component.

### (Supplementary Note 2)

The vasodilator composition according to Supplementary Note 1, wherein
a microbubble density is 5×10⁵ bubbles/ml to 5×10¹² bubbles/ml.

### (Supplementary Note 3)

The vasodilator composition according to Supplementary Note 1 or 2, wherein
in the gas component, a proportion of the nitric oxide is 80% or more.

### (Supplementary Note 4)

The vasodilator composition according to any one of Supplementary Notes 1 to 3, wherein
the gas component is substantially free of oxygen.

### (Supplementary Note 5)

The vasodilator composition according to any one of Supplementary Notes 1 to 4, further including:
a medium, wherein
the medium is at least one of a liquid or a solid.

### (Supplementary Note 6)

A vasodilator composition kit, including:
a vasodilator composition; and
other component, wherein
the vasodilator composition and the other component are arranged in isolation, and
the vasodilator composition is the vasodilator composition according to any one of Supplementary Notes 1 to 5.

### (Supplementary Note 7)

The vasodilator composition kit according to Supplementary Note 6, further including:
a container, wherein
the container includes a first chamber, a second chamber, and an isolation portion, wherein
the vasodilator composition is contained in the first chamber,
the other component is contained in the second chamber, and
the isolation portion isolates the first chamber from the second chamber and allows the first chamber and the second chamber to communicate with each other.

### (Supplementary Note 8)

The vasodilator composition kit according to Supplementary Note 6 or 7, wherein
the other component includes an osmotic regulator.

### (Supplementary Note 9)

A pharmaceutical composition for a disease caused by a vascular stenosis or vascular occlusive disorder, including:
the vasodilator composition according to any one of Supplementary Notes 1 to 5.

### (Supplementary Note 10)

The pharmaceutical composition according to Supplementary Note 9, wherein
the disease caused by a vascular stenosis or vascular occlusive disorder is at least one selected from the group consisting of angina pectoris, myocardial infarction, cerebral infarction, transient cerebral ischemic attack, pulmonary hypertension, and acute heart failure.

### (Supplementary Note 11)

The pharmaceutical composition according to Supplementary Note 9 or 10, wherein
the disease caused by a vascular stenosis or vascular occlusive disorder is angina pectoris, and
the pharmaceutical composition is for use in prevention, suppression, reduction, improvement, remission, or amelioration of an angina attack.

### (Supplementary Note 12)

The pharmaceutical composition according to any one of Supplementary Notes 9 to 11 for intravenous administration.

### (Supplementary Note 13)

A pharmaceutical kit for a disease caused by a vascular stenosis or vascular occlusive disorder, including:
the vasodilator composition kit according to any one of Supplementary Notes 6 to 8.

### (Supplementary Note 14)

The pharmaceutical kit according to Supplementary Note 13, wherein
the disease caused by a vascular stenosis or vascular occlusive disorder is at least one selected from the group consisting of angina pectoris, myocardial infarction, cerebral infarction, transient cerebral ischemic attack, pulmonary hypertension, and acute heart failure.

### (Supplementary Note 15)

The pharmaceutical kit according to Supplementary Note 13 or 14, wherein
the disease caused by a vascular stenosis or vascular occlusive disorder is angina pectoris, and
the pharmaceutical kit is for use in prevention, suppression, reduction, improvement, remission, or amelioration of an angina attack.

### (Supplementary Note 16)

The pharmaceutical kit according to any one of Supplementary Notes 13 to 15 for use in intravenous administration.

### (Supplementary Note 17)

A method for treating a disease caused by a vascular stenosis or vascular occlusive disorder, including the step of:
administering to a patient the vasodilator composition according to any one of Supplementary Notes 1 to 5.

### (Supplementary Note 18)

A method for treating a disease caused by a vascular stenosis or vascular occlusive disorder, including the steps of:
mixing a vasodilator composition and other component in the vasodilator composition kit according to any one of Supplementary Notes 6 to 8, and
administering to a patient a resulting mixture.

### (Supplementary Note 19)

The treatment method according to Supplementary Note 17 or 18, wherein
the disease caused by a vascular stenosis or vascular occlusive disorder is at least one selected from the group consisting of angina pectoris, myocardial infarction, cerebral infarction, transient cerebral ischemic attack, pulmonary hypertension, and acute heart failure.

### (Supplementary Note 20)

The treatment method according to Supplementary Note 18 or 19, wherein
the disease caused by a vascular stenosis or vascular occlusive disorder is angina pectoris, and
administration of the vasodilator composition and the resulting mixture allows prevention, suppression, reduction, improvement, remission, or amelioration of an angina attack.

### (Supplementary Note 21)

The treatment method according to any one of Supplementary Notes 17 to 20, wherein
the vasodilator composition or mixture is administered intravenously.

### (Supplementary Note 22)

A vasodilator composition for use in vasodilation, including:
microbubbles, wherein
the microbubbles contain nitric oxide as a gas component.

### (Supplementary Note 23)

A vasodilator composition for use in a treatment of a disease caused by a vascular stenosis or vascular occlusive disorder, including:
microbubbles, wherein
the microbubbles contain nitric oxide as a gas component.

### (Supplementary Note 24)

The vasodilator composition according to Supplementary Note 23, wherein
the disease caused by a vascular stenosis or vascular occlusive disorder is at least one selected from the group consisting of angina pectoris, myocardial infarction, cerebral infarction, transient cerebral ischemic attack, pulmonary hypertension, and acute heart failure.

### (Supplementary Note 25)

The vasodilator composition according to Supplementary Note 23 or 24, wherein
the disease caused by a vascular stenosis or vascular occlusive disorder is angina pectoris, and
administration of the vasodilator composition and the resulting mixture allows prevention, suppression, reduction, improvement, remission, or amelioration of an angina attack.

### (Supplementary Note 26)

The vasodilator composition according to any one of Supplementary Notes 23 to 25 for use in intravenous administration.

### Industrial Applicability

As described above, according to the present invention, the dosage of NO can be adjusted. Further, according to the composition of the present invention, since the dosage of NO can be adjusted, it is also possible to adjust, for example, the degree of vasodilation. In addition, since the composition of the present invention can be administered directly into the body of the subject of administration, for example, local administration as well as systemic administration can be performed as in inhalation administration, for example. Thus, the present invention can be suitably used, for example, for the treatment of diseases caused by a vascular stenosis, and is extremely useful in the medical field, the pharmaceutical field, and the like.

### Reference Signs List

1: first chamber
10: container
11: composition
12: upper-end portion
13, 14: sheet
2: second chamber
21: other component
22: discharge portion
3: isolation portion
5: hanging portion

## Claims

1. A vasodilator composition, comprising:
microbubbles, wherein
the microbubbles contain nitric oxide as a gas component.

2. The vasodilator composition according to claim 1, wherein
a microbubble density is 5×10⁵ bubbles/ml to 5×10¹² bubbles/ml.

3. The vasodilator composition according to claim 1 or 2, wherein
in the gas component, a proportion of the nitric oxide is 80% or more.

4. The vasodilator composition according to any one of claims 1 to 3, wherein
the gas component is substantially free of oxygen.

5. The vasodilator composition according to any one of claims 1 to 4, further comprising:
a medium, wherein
the medium is at least one of a liquid or a solid.

6. A vasodilator composition kit, comprising:
a vasodilator composition; and
other component, wherein
the vasodilator composition and the other component are arranged in isolation, and
the vasodilator composition is the vasodilator composition according to any one of claims 1 to 5.

7. The vasodilator composition kit according to claim 6, further comprising:
a container, wherein
the container includes a first chamber, a second chamber, and an isolation portion, wherein
the vasodilator composition is contained in the first chamber,
the other component is contained in the second chamber, and
the isolation portion isolates the first chamber from the second chamber and allows the first chamber and the second chamber to communicate with each other.

8. The vasodilator composition kit according to claim 6 or 7, wherein
the other component includes an osmotic regulator.

9. A pharmaceutical composition for a disease caused by a vascular stenosis or vascular occlusive disorder, comprising:
the vasodilator composition according to any one of claims 1 to 5.

10. The pharmaceutical composition according to claim 9, wherein
the disease caused by a vascular stenosis or vascular occlusive disorder is at least one selected from the group consisting of angina pectoris, myocardial infarction, cerebral infarction, transient cerebral ischemic attack, pulmonary hypertension, and acute heart failure.

11. The pharmaceutical composition according to claim 9 or 10, wherein
the disease caused by a vascular stenosis or vascular occlusive disorder is angina pectoris, and
the pharmaceutical composition is for use in prevention, suppression, reduction, improvement, remission, or amelioration of an angina attack.

12. The pharmaceutical composition according to any one of claims 9 to 11 for intravenous administration.

13. A pharmaceutical kit for a disease caused by a vascular stenosis or vascular occlusive disorder, comprising:
the vasodilator composition kit according to any one of claims 6 to 8.

14. The pharmaceutical kit according to claim 13, wherein
the disease caused by a vascular stenosis or vascular occlusive disorder is at least one selected from the group consisting of angina pectoris, myocardial infarction, cerebral infarction, transient cerebral ischemic attack, pulmonary hypertension, and acute heart failure.

15. The pharmaceutical kit according to claim 13 or 14, wherein
the disease caused by a vascular stenosis or vascular occlusive disorder is angina pectoris, and
the pharmaceutical kit is for use in prevention, suppression, reduction, improvement, remission, or amelioration of an angina attack.

16. The pharmaceutical kit according to any one of claims 13 to 15 for use in intravenous administration.
